# FASCICULE DE BREVET EUROPEEN

(11) **EP 0 770 391 B1**
(45) Date de publication et mention de la délivrance du brevet: **02.12.1998**
(21) Numéro de dépôt: 96402181.0
(22) Date de dépôt: 14.10.1996
(51) Int. Cl.: A61K 33/30, A61K 33/32, A61K 33/24, A61K 33/00

(54) **Utilisation de sel de lanthanide, de manganèse, d'étain, de zinc ou d'yttrium, de cobalt, de baryum, de strontium dans des compositions pharmaceutiques**
Verwendung eines Lanthanoid-, Zinn-, Mangan-, Yttrium-, Kobalt-, Barium-, Strontiumsalzes für die Herstellung einer pharmazeutischen Zusammensetzung
Use of salts of lanthanide, tin, zinc, manganese, yttrium, cobalt, strontium in pharmaceutical compositions

(30) Priorité: 26.10.1995 FR 9512657
(43) Date de publication de la demande: 02.05.1997
(73) Titulaire: L'OREAL, 75008 Paris (FR)
(72) Inventeur: de Lacharriere, Olivier, 75015 Paris (FR); Breton, Lionel, 78000 Versailles (FR)
(74) Mandataire: Lhoste, Catherine

(56) Documents cités:
- EP-A- 0 068 854
- EP-A- 0 085 579
- EP-A- 0 093 538
- EP-A- 0 135 312
- EP-A- 0 234 733
- DE-A- 1 935 879
- FR-M- 5 394
- GB-A- 2 133 285

## Description

La présente invention se rapporte à l'utilisation d'au moins un sel de lanthanide, de manganèse, d'étain, de zinc ou d'yttrium, de cobalt, de baryum, de strontium pour la préparation d'une composition pharmaceutique pour traiter l'un au moins des symptômes associés aux différentes affections et/ou troubles suivants: l'emphysème, la toux, les gastro-entérites, la gastrite, les troubles de la spasticite intestinale, l'incontinence vésicale, la cystite, les scléroses multiformes, la maladie de Parkinson, la maladie d'Alzheimer, le syndrome de Korsakoff, l'ulcère gastrique, la maladie de Raynaud.

Il existe chez les mammifères des polypeptides appartenant à la famille des tachykinines qui induisent sur les fibres musculaires lisses des contractions rapides. Parmi les composés de cette famille on peut citer la neurokinine β, neurokinine α et la substance P.

La substance P est un élément chimique polypeptidique (undécapeptide), élaboré et libéré par une terminaison nerveuse. La localisation de la substance P est spécifique des neurones, tant dans le système nerveux central que dans les organes à la périphérie. Ainsi, de très nombreux organes ou tissus reçoivent des afférences de neurones à substance P, il s'agit notamment des glandes salivaires, de l'estomac, du pancréas, de l'intestin (dans celui-ci, la distribution de la substance P est superposée au plexus nerveux intrinsèque de Meissner et d'Auerbach), du système cardio-vasculaire, de la glande thyroïde, de la peau, de l'iris et des corps ciliaires, de la vessie et bien évidemment des systèmes nerveux central et périphérique.

De par la distribution ubiquitaire de la substance P, de très nombreux désordres sont associés à un excès de synthèse et / ou de libération de substance P.

La substance P intervient notamment dans la transmission de la douleur (dentaire cutané, tympanique) et dans des maladies du système nerveux central (par exemple l'anxiété, les psychoses, les neuropathies, les troubles neurodégénératifs de type démence sénile d'Alzheimer, démence des sidéens, maladie de Parkinson, syndrome de Down, syndrome de Korsakoff, scléroses multiples, schizophrénie, maladies psychotiques), dans des maladies respiratoires (telles que par exemple les broncho-pneumonie, toux, emphysème, bronchiolite) et inflammatoires (telles que par exemple la polyarthrite rhumatoïde), dans des syndromes allergiques (tels que par exemple l'asthme, les rhinites allergiques, les pharyngites allergiques, l'urticaire, les dermatites eczémateuses), dans des maladies gastro-intestinales (telles que par exemple les ulcères, les colites, la maladie de Crohn, gastrites, gastro-entérites, spasticites intestinales), dans des troubles cardio-vasculaires, des troubles vasospastiques (tels que par exemple les migraines, la maladie de Raynaud), dans des désordres immunologiques, dans des troubles du tractus urinaire et ou génital (tels que par exemple l'incontinence, la cystite), dans des maladies rhumatismales dans certaines maladies dermatologiques (telles que l'eczéma) et dans les affections ophtalmologiques (telles que par exemple les conjonctivites, les uvéites, les prurits oculaires, les blépharites, les douleurs oculaires et les irritations).

L'utilisation d'antagoniste de substance P est l'une des alternatives thérapeutiques efficaces dans toutes les affections citées ci-dessus.

Par antagoniste de substance P, on entend tout composé susceptible d'inhiber partiellement, voire totalement, l'effet biologique de la substance P. Particulièrement, pour qu'une substance soit reconnue comme un antagoniste de substance P elle doit induire une réponse pharmacologique cohérente (incluant ou non sa fixation au récepteur de la substance P) notamment dans l'un des tests suivants :
- la substance antagoniste doit diminuer l'extravasation du plasma au travers de la paroi vasculaire induite par la capsaïcine ou par une stimulation nerveuse antidromique, ou bien
- la substance antagoniste doit provoquer une inhibition de la contraction des muscles lisses induites par l'administration de substance P.

A ce jour, des antagonistes de substance P sont utilisés pour traiter les désordres indiqués ci-dessus et sont cités dans de nombreux documents.

Cependant aucun de ces documents n'envisage, ni ne suggère qu'un sel de lanthanide, de zinc, d'yttrium, d'étain, de cobalt, de baryum, de strontium puisse avoir une activité antagoniste de substance P telle que définie ci-dessus et donc puisse être notamment utilisé pour traiter les douleurs associées aux symptômes associés aux différentes affections et/ou troubles indiqués ci-dessus.

En outre, il est connu du document FR-M-5394 un médicament contenant des crésols et du chlorure de strontium pour augmenter les propriétés antibactériennes et antimicrobiennes de ces crésols. Toutefois, ce document ne décrit ni ne suggère que le chlorure de strontium puisse diminuer voire éliminer, à lui seul, les symptômes tels que les irritations, douleurs, démangeaisons, picotements, causés par une quelconque maladie, ni diminuer voire supprimer ces mêmes symptômes causés par un produit irritant.

La demanderesse a découvert qu'un sel choisi parmi les sels de lanthanide, de manganèse, d'étain, de zinc, d'yttrium, de cobalt, de baryum, de strontium et leurs associations répond aux caractéristiques définies comme caractérisant un antagoniste de substance P.

L'invention concerne l'utilisation d'au moins un sel choisi parmi les sels de lanthanide, de manganèse, d'étain, de zinc, d'yttrium, de cobalt, de baryum, de strontium et leurs associations pour la préparation d'une composition pharmaceutique destinée à traiter l'une au moins des symptômes associés aux différentes affections et/ou troubles suivants : l'emphysème, la toux, les gastro-entérites, la gastrite, les troubles de la spasticite intestinale, l'incontinence vésicale, la cystite, les scléroses multiformes, la maladie de Parkinson, la maladie d'Alzheimer, le syndrome de Korsakoff, l'ulcère gastrique, la maladie de Raynaud.

Par lanthanide, on entend les éléments de numéro atomique z allant de 57 à 71, c'est-à-dire le lanthane, le cérium, le praséodyme, le néodyme, le prométhium, le samarium, l'europium, le gadolinium, le terbium, le dysprosium, l'holmium, l'erbium, le thulium, l'ytterbium, le lutétium.

L'invention s'applique à tous les désordres liés à un excès de synthèse et/ou de libération de substance P cités précédemment.

Les sels utilisés peuvent être par exemple des carbonates, des bicarbonates, des sulfates, des glycérophosphates, des borates, des chlorures, des nitrates, des acétates, des hydroxydes, des persulfates ainsi que des sels d'a-hydroxyacides (citrates, tartrates, lactates, malates) ou d'acides de fruits, ou encore des sels d'acides aminés (aspartate, arginate, glycocholate, fumarate) ou des sels d'acides gras (palmitate, oléate, caséinate, béhénate).

De préférence, on utilise des chlorures et des nitrates avantageusement de zinc, de gadolinium, d'yttrium et de strontium.

Les sels peuvent être choisi parmi les sels de zinc, de baryum, de manganèse, d'yttrium, de strontium et leurs associations.

De façon préférée, le sel est un sel de strontium.

Selon l'invention, le sel peut être utilisé en une quantité allant de 10⁻⁵ % à 20 % du poids total de la composition et préférentiellement en une allant de 10⁻² % à 15 % du poids total de la composition et mieux de 0,5 à 8% du poids total de la composition.

Le sel peut être utilisé dans une composition qui doit être ingérée, injectée localement, instillée ou destinée au lavage. Selon le mode d'administration, cette composition peut se présenter sous toutes les formes galéniques normalement utilisées.

Pour une ingestion, la composition peut avoir la forme notamment de solution aqueuse hydroalcoolique ou huileuse ou de suspension huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, de microémulsions ou encore de microcapsules ou microparticules, ou de dispersions vésiculaires de type ionique et/ou non ionique. Ces compositions sont préparées selon les méthodes usuelles.

Pour l'injection locale, l'instillation ou le lavage, la composition peut se présenter sous forme de lotion aqueuse, de suspension huileuse ou sous forme de sérum. Pour l'ingestion, elle peut se présenter sous forme de capsules, de granulés de sirops ou de comprimés.

Les quantités des différents constituants des compositions selon l'invention sont celles classiquement utilisées dans les domaines considérés.

Les compositions peuvent aussi être conditionnées sous forme de composition pour aérosol comprenant également un agent propulseur sous pression.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition pharmaceutique peut contenir également des adjuvants habituels dans le domaine pharmaceutique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine pharmaceutique, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras, des acides gras (acide stéarique), des cires d'abeilles, de carnauba, et de la paraffine.

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux ou bactériens et l'amidon.

Comme actifs lipophiles, on peut utiliser le rétinol (vitamine A) et ses dérivés, le tocophérol (vitamine E) et ses dérivés, les acides gras essentiels, les céramides, les huiles essentielles.

Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxy-carboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle
- les antiseptiques
- les antimétabolites.

De façon avantageuse, selon l'invention au moins un sel de lanthanide, de manganèse, d'étain, de zinc, d'yttrium, de cobalt, de baryum, de strontium peut être associé à des produits à effet irritant utilisés couramment dans le domaine pharmaceutique, produits qui sont parfois des actifs pharmaceutiques. La présence d'un antagoniste de substance P sous la forme d'au moins un sel de lanthanide, de manganèse, d'étain, de zinc, d'yttrium, de cobalt, de baryum, de strontium pour la fabrication d'une composition pharmaceutique comprenant un produit ayant un effet irritant permet d'atténuer fortement, voire de supprimer cet effet irritant.

Cela permet en outre d'augmenter la quantité d'actif à effet irritant par rapport à la quantité d'actif normalement utilisée, en vue d'une efficacité améliorée.

La présente invention a aussi pour objet un procédé de traitement thérapeutique des symptômes associés aux différentes affections et/ou troubles suivants : l'emphysème, la toux, les gastro-entérites, la gastrite, les troubles de la spasticite intestinale, l'incontinence vésicale, la cystite, les scléroses multiformes, la maladie de Parkinson, la maladie d'Alzheimer, le syndrome de Korsakoff, l'ulcère gastrique, la maladie de Raynaud consistant à injecter, ingérer, instiller, appliquer une composition telle que définie précédemment.

Les exemples et compositions suivants illustrent l'invention sans la limiter aucunement. Dans les compositions les proportions indiquées sont des pourcentages en poids.

### EXEMPLE 1 : Solution buvable

- - Chlorure de strontium: 0,5
- - Excipient :: qsp 100,00

Chlorure de sodium
Borate de sodium
Polysorbate 80
Acide borique
Eau

### EXEMPLE 2 : Solution injectable localement

- - Aspartate de baryum: 1,00
- - Excipient: qsp 100,00

Chlorure de sodium
Glucose
Eau

### EXEMPLE 3 : Solution buvable

- - Chlorure de baryum: 2,00
- - Excipient: qsp 100,00

Acide borique
Chlorure de sodium
Eau

## Revendications

1. Utilisation d'au moins un sel choisi parmi les sels d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, d'étain, de zinc, de manganèse, de cobalt, de baryum ou de strontium pour la fabrication d'une composition pharmaceutique pour traiter l'un au moins des symptômes associés aux différentes affections et/ou troubles suivants : l'emphysème, la toux, les gastro-entérites, la gastrite, les troubles de la spasticite intestinale, l'incontinence vésicale, la cystite, les scléroses multiformes, la maladie de Parkinson, la maladie d'Alzheimer, le syndrome de Korsakoff, l'ulcère gastrique, la maladie de Raynaud.

2. Utilisation selon la revendication 1, caractérisée en ce que les sels sont constitués d'ions choisis parmi les ions chlorure, borate, bicarbonate, carbonate, nitrate, hydroxyde, sulfate, glycérophosphate, les acides de fruit, les acide aminés.

3. Utilisation selon l'une des revendications précédentes, caractérisée en ce que la sel est choisi parmi les sels de zinc, de baryum, de manganèse, d'yttrium, de strontium et leur associations.

4. Utilisation selon l'une des revendications précédentes, caractérisée en ce que le sel est un sel de strontium.

5. Utilisation selon l'une des revendications précédentes, caractérisée en ce que les ions sont les ions chlorures, nitrates.

6. Utilisation selon l'une quelconque des revendications précédentes, caractérisée en ce que le sel est utilisé en une quantité allant de 10⁻⁵ % à 20 % du poids total de la composition.

7. Utilisation selon l'une des revendications précédentes, caractérisée en ce que le sel est utilisé en une quantité représentant de 10⁻² % à 15 % du poids total de la composition.

8. Utilisation selon l'une des revendications précédentes, caractérisée en ce que la composition comprend de plus au moins un actif pharmaceutique différent des sels.

9. Utilisation selon la revendication 8, caractérisée en ce que la composition contient, en outre, au moins un actif choisi parmi un agent choisi parmi les agents antibactériens, antiparasitaires, antifongiques, antiviraux anti-inflammatoires, antiprurigineux, anesthésiques, kératolytiques, anti-radicaux libres, anti-séborrhéiques, antipelliculaires, antiacnéiques et/ou les agents modulant la différenciation et/ou la prolifération et/ou la pigmentation cutanée.

10. Utilisation selon l'une des revendications précédentes, caractérisée en ce que la composition se présente sous forme d'une solution aqueuse, huileuse, hydroalcoolique, d'une émulsion huile-dans-eau ou eau-dans-huile, d'une microémulsion, d'un gel aqueux anhydre, d'un sérum, d'une dispersion de vésicules.

## Claims

1. Use of at least one salt chosen from yttrium, lanthanum, cerium, praseodymium, neodymium, promethium, samarium, europium, gadolinium, terbium, dysprosium, holmium, erbium, thulium, ytterbium, lutetium, tin, zinc, manganese, cobalt, barium or strontium salts for the manufacture of a pharmaceutical composition for treating at least one of the symptoms associated with the various conditions and/or disorders which follow: emphysema, cough, gastro-enteritis, gastritis, intestinal spasticity disorders, vesical incontinence, cystitis, multiple scleroses, Parkinson's disease, Alzheimer's disease, Korsakoff's syndrome, gastric ulcer, Raynaud's disease.

2. Use according to Claim 1, characterized in that the salts consist of ions chosen from chloride, borate, bicarbonate, carbonate, nitrate, hydroxide, sulphate, and glycerophosphate ions, fruit acids, amino acids.

3. Use according to one of the preceding claims, characterized in that the salt is chosen from zinc, barium, manganese, yttrium or strontium salts and combinations thereof.

4. Use according to one of the preceding claims, characterized in that the salt is a strontium salt.

5. Use according to one of the preceding claims, characterized in that the ions are chloride or nitrate ions.

6. Use according to any one of the preceding claims, characterized in that the salt is used in a quantity ranging from 10⁻⁵ % to 20 % of the total weight of the composition.

7. Use according to one of the preceding claims, characterized in that the salt is used in a quantity representing from 10⁻² % to 15 % of the total weight of the composition.

8. Use according to one of the preceding claims, characterized in that the composition comprises, in addition, at least one pharmaceutical active agent different from the salts.

9. Use according to Claim 8, characterized in that the composition contains, in addition, at least one active agent chosen from an agent chosen from antibacterial, antipara- sitic, antifungal, antiviral, anti-inflammatory, antipruritic, anaesthetic, keratolytic, anti-free radical, antiseborrhoeic, anti-dandruff, or anti-acne agents and/or skin pigmentation and/or proliferation and/or differentiation modulating agents.

10. Use according to one of the preceding claims, characterized in that the composition is provided in the form of an aqueous, oily or aqueous-alcoholic solution, an oil-in-water or water-in-oil emulsion, a micro-emulsion, an aqueous or anhydrous gel, a serum, or a dispersion of vesicules.

## Patentansprüche

1. Verwendung mindestens eines Salzes, das unter Yttrium-, Lanthan-, Cer-, Praseodym-, Neodym-, Promethium-, Samarium-, Europium-, Gadolinium-, Terbium-, Dysprosium-, Holmium-, Erbium-, Thulium-, Ytterbium-, Lutetium-, Zinn-, Zink-, Mangan-, Cobalt-, Barium- und Strontium-Salzen ausgewählt wird, für die Herstellung einer pharmazeutischen Zusammensetzung zur Behandlung mindestens eines der mit den verschiedenen folgenden Krankheiten und/oder Störungen einhergehenden Symptome: Emphysem, Husten, Gastroenteritis, Gastritis, Darmspastizitäts-Störungen, Blaseninkontinenz, Cystitis, vielfältige Sklerosen, Parkinson-Krankheit, Alzheimer-Krankheit, Korsakoff-Syndrom, Magengeschwür, Raynaud-Krankheit.

2. Verwendung nach Anspruch 1, dadurch gekennzeichnet, daß die Salze aus Ionen bestehen, die unter Chlorid-, Borat-, Hydrogencarbonat-, Carbonat-, Nitrat-, Hydroxid-, Sulfat-, Glycerophosphat-Ionen, den Ionen von Fruchtsäuren und den Ionen von Aminosäuren ausgewählt werden.

3. Verwendung nach einem der vorhergehenden Ansprüche, dadurch gekennzeichnet, daß das Salz unter Zink-, Barium-, Mangan-, Yttrium-, Strontium-Salzen und deren Kombinationen ausgewählt werden.

4. Verwendung nach einem der vorhergehenden Ansprüche, dadurch gekennzeichnet, daß das Salz ein Strontiumsalz ist.

5. Verwendung nach einem der vorhergehenden Ansprüche, dadurch gekennzeichnet, daß die Ionen Chloridionen, Nitrationen sind.

6. Verwendung nach einem der vorhergehenden Ansprüche, dadurch gekennzeichnet, daß das Salz in einer Menge von 10⁻⁵ bis 20 % des Gesamtgewichts der Zusammensetzung verwendet wird.

7. Verwendung nach einem der vorhergehenden Ansprüche, dadurch gekennzeichnet, daß das Salz in einer Menge von 10⁻² bis 15 % des Gesamtgewichts der Zusammensetzung verwendet wird.

8. Verwendung nach einem der vorhergehenden Ansprüche, dadurch gekennzeichnet, daß die Zusammensetzung außerdem mindestens einen von den Salzen verschiedenen pharmazeutischen Wirkstoff enthält.

9. Verwendung nach Anspruch 8, dadurch gekennzeichnet, daß die Zusammensetzung außerdem mindestens einen Wirkstoff enthält, der unter antibakteriellen Mitteln, antiparasitären Mitteln, fungizid wirksamen Mitteln, antiviralen Mitteln, entzündungshemmenden Mitteln, Antipuriginosa, Anasthetika, Keratolytika, Mitteln gegen freie Radikale, Antiseborrhöika, Antischuppenmitteln, Aknemitteln und/oder Mitteln, die die Differenzierung und/oder die Proliferation und/oder die Pigmentierung der Haut modulieren, ausgewählt wird.

10. Verwendung nach einem der vorhergehenden Ansprüche, dadurch gekennzeichnet, daß die Zusammensetzung in Form einer wäßrigen, öligen, wäßrig-alkoholischen Lösung, einer Öl-in-Wasser-Emulsion oder einer Wasserin-Öl-Emulsion, einer Mikroemulsion, eines wasserfreien Gels, eines Serums, einer Vesikeldispersion vorliegt.
